(19) Europäisches Patentamt
European Patent Office
Office européen des brevets

(11) EP 0 395 313 B1

(12) **EUROPEAN PATENT SPECIFICATION**

(45) Date of publication and mention of the grant of the patent:
**15.12.1999 Bulletin 1999/50**

(51) Int Cl.[6]: **C07D 295/08**, C07D 295/12, C07D 295/14, A61K 31/495, C07D 239/42

(21) Application number: **90304251.3**

(22) Date of filing: **20.04.1990**

(54) **Tertiary alkyl functionalized piperazine derivatives**

Tertiäre alkyl funktionalisierte Piperazin-Derivate

Dérivés de pipérazine avec une fonction alkyle tertiaire

(84) Designated Contracting States:
**AT BE CH DE DK ES FR GR IT LI LU NL SE**

(30) Priority: **22.04.1989 GB 8909209**
**27.10.1989 US 428148**

(43) Date of publication of application:
**31.10.1990 Bulletin 1990/44**

(60) Divisional application: **99108070.6 / 0 955 296**

(73) Proprietors:
- **AMERICAN HOME PRODUCTS CORPORATION Madison, New Jersey 07940-0874 (US)**
- **JOHN WYETH & BROTHER LIMITED Maidenhead Berkshire, SL6 0PH (GB)**

(72) Inventors:
- **Cliffe, Ian Anthony Cippenham, Slough, Berkshire (GB)**
- **Abou-Gharbia, Magid Abdel-Megid Glen Mills, Pennsylvania (US)**
- **Yardley, John Patrick Gulph Mills, Pennsylvania (US)**

(74) Representative: **Brown, Keith John Symons et al c/o Wyeth Laboratories Huntercombe Lane South Taplow Maidenhead Berkshire SL6 0PH (GB)**

(56) References cited:
**EP-A- 0 015 615**
**EP-A- 0 048 045**
**EP-A- 0 238 905**
**GB-A- 2 155 925**
**US-A- 4 202 898**
**US-A- 4 797 489**

EP 0 395 313 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to novel tertiary alkyl functional piperazine derivatives, to processes for their preparation, to their use and to pharmaceutical compositions containing them.

The anxiolytic activity of buspirone and structurally related compounds has been attributed to their selective activity at a serotonin (5-hydroxytyptamine; 5-HT) subtype receptor designated the $5\text{-}HT_{1A}$ receptor. US-A-4,202,898 discloses the treatment of anxiety and depression with a group of substituted phenylpiperazine derivatives. Among the compounds disclosed as useful for that purpose is 2,2-2,2-dimethylpropanoic acid (3-trifluoromethylthio phenyl-piperazino)ethyl ester (column 3, compound 21). Related compounds are disclosed in FR-A-2432516. The therapeutic value of $5\text{-}HT_{1A}$ receptor agonists in treating multi-CNS disorders has recently been extended to compound structures such as umespirone which has high affinity for both the $5\text{-}HT_{1A}$ and $D_2$ receptor binding sites (DE-A-3529872). US-A-4,797,489 by Abou-Gharbia et al discloses adamantyl- and fluorenyl-arylpiperazines and -arylpiperidines which are useful for the treatment of CNS disorders.

EP-A-0318933 discloses substituted piperazines which are acyclic amides and which are useful as antipsychotic and/or anxiolytic agents. Further substituted piperazines are disclosed in EP-A-0015615 and GB-A-2155925 as having anti-agressive and antipsychotic properties respectively. 4-(2-Pyrimidinyl)-1-piperazinyl heterocyclic carbonyl derivatives having anxiolytic properties are disclosed in EP-A-0238905.

In accordance with this invention there is provided a group of novel compounds which exhibit serotonin $5\text{-}HT_{1A}$ receptor affinity which characterizes them as antidepressant and/or anxiolytic agents. Some of the compounds of this invention exhibit both $5\text{-}HT_{1A}$ receptor affinity and dopamine $D_2$ receptor binding which characterizes them as anxiolytic and/or antidepressant agents with elements of antipsychotic activity. The compounds of this invention are of the following structural formula:

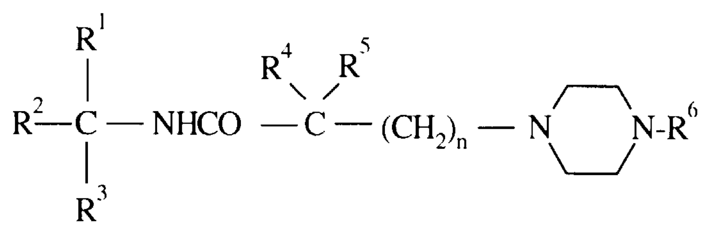

in which

| | |
|---|---|
| $R^1$ | is alkyl of 1 to 6 carbon atoms; |
| $R^2$ and $R^3$ | are alkyl of 1 to 6 carbon atoms or taken together they are polymethylene of 2 to 12 carbon atoms; |
| $R^4$ | is hydrogen or alkyl of 1 to 6 carbon atoms; |
| $R^5$ | is hydrogen, alkyl of 1 to 8 carbon atoms, hydroxyalkyl of 1 to 3 carbon atoms, phenyl, benzyl, substituted phenyl or substituted benzyl in which the substituents are hydroxy, halo, alkyl of 1 to 6 carbon atoms, alkoxy of 1 to 6 carbon atoms, trifluoromethyl, nitro, cyano, carbalkoxy of 2 to 7 carbon atoms, carboxamido, amino, alkylamino of 1 to 6 carbon atoms or dialkylamino of 2 to 12 carbon atoms; |
| $R^6$ | is phenyl, benzyl, 2-, 3-, or 4-pyridinyl, 2-pyrimidinyl or 2-pyrazinyl; any of which may be substituted by one or more hydroxy, halo, alkyl of 1 to 6 carbon atoms, alkoxy of 1 to 6 carbon atoms, trifluoromethyl, nitro, cyano, carbalkoxy of 2 to 7 carbon atoms, carboxamido, amino, alkylamino of 1 to 6 carbon atoms or dialkylamino of 2 to 12 carbon atoms; and |
| n | is one of the integers 1 or 2; or a pharmaceutically acceptable salt thereof. |

The preferred compounds from the standpoint of production economics and activity profile are those of the formula indicated above

wherein

| | |
|---|---|
| $R^1$ | is alkyl of 1 to 6 carbon atoms; |
| $R^2$ and $R^3$ | are alkyl of 1 to 6 carbon atoms or taken together they are polymethylene of 2 to 6 carbon atoms; |
| $R^4$ | is hydrogen or alkyl of 1 to 6 carbon atoms; |
| $R^5$ | is hydrogen, alkyl of 1 to 6 carbon atoms, hydroxyalkyl of 1 to 3 carbon atoms, phenyl, benzyl, p-hydroxyphenyl, p-methoxyphenyl, o-methoxyphenyl, p-chlorophenyl or p-fluorophenyl; |
| $R^6$ | is phenyl, 2-alkylphenyl in which the alkyl substituent has 1 to 6 carbon atoms, 2-alkoxyphenyl in which the alkoxy substituent has 1 to 6 carbon atoms, 2-halophenyl, 2-cyanophenyl, 2-nitrophenyl, 2-perhalomethylphenyl, benzyl, 2-, 3-, or 4-pyridinyl, 2-pyrimidinyl or 2-pyrazinyl; and |
| n | is one of the integers 1 or 2; or a pharmaceutically acceptable salt thereof. |

The halo substituent referred to in the preceding paragraph may be chloro-, bromo-, fluoro- or iodo, the chloro-, bromo- and fluoro- substituents being preferred.
For simplicity, the lower alkyl and alkoxy groups containing 1 to 4 carbon atoms are preferred throughout the molecules. The pharmaceutically acceptable salts are produced conventionally from such acids as hydrochloric, hydrobromic, sulfuric, phosphoric, methane sulfonic, nitric, p-toluene sulfonic, acetic, citric and maleic, succinic acid.
The compounds of this invention present chiral centers depending upon the substituent variations of $R^1$, $R^2$ and $R^3$ and $R^4$ and $R^5$. The optical isomers generated at these positions may be separated and isolated or directly produced from reactants of known or related configurations, by conventional means.
Highly selective $5HT_{1A}$ receptor binding has been observed with the compounds of this invention in which $R^5$ is alkyl of 4 to 8 carbon atoms or an optionally substituted phenyl or benzyl moiety. These compounds demonstrate only poor relative binding to the $D_2$ and $\alpha_1$ receptors. As such, their selective anxiolytic activity is clearly indicated.
The compounds of this invention are produced by various conventional methods from commercially available starting materials or reactants producible by conventional techniques.
In one method for preparing the compounds of the invention an amine or the formula:

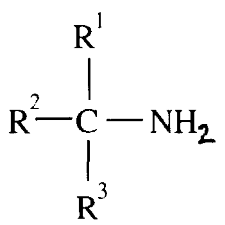

is acylated with an acid of the formula:

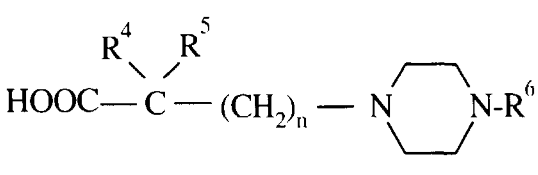

or with any acylating derivative thereof. Examples of acylating derivatives include the acid halides (eg. acid chlorides), azides, anhydrides, imidazolides (eg obtained from carbonyldiimidazole), activated esters or O-acyl ureas obtained from a carbodiimide such as a dialkylcarbodiimide particularly dicyclohexylcarbodiimide. Preferably the amine is acylated with the acid in presence of a coupling agent such as 1,1'-carbonyldiimidazole, iso-butylchloroformate or diphenylphosphinyl chloride.
An alternative method of preparing the compounds of the invention comprises alkylation of a piperazine of the formula:

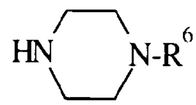

with an alkylating agent providing the group:

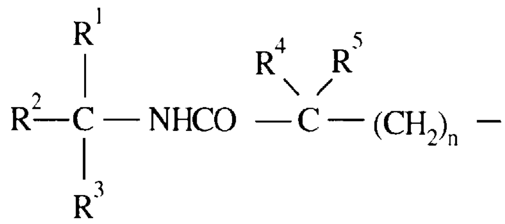

The alkylating agent may be, for example, a compound of the formula:

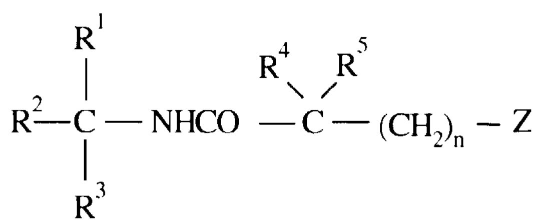

where Z is a leaving group such as halogen or an alkyl- or aryl-sulphonyloxy group. Alternatively where n is 1 the alkylating agent may be an unsaturated compound of the formula:

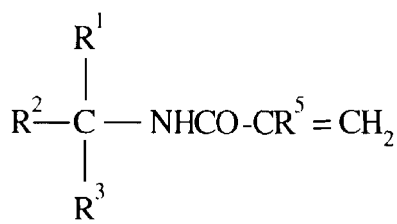

and the compounds are reacted by means of a Michael reaction. The reaction may be carried out at elevated temperature in the presence of an alcohol.

The compounds of the invention may also be prepared by an alternative method comprising reacting a nitrile of the formula:

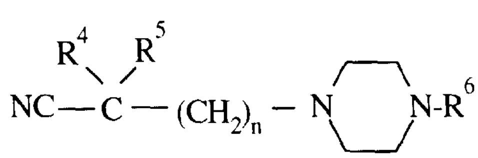

with the tertiary alcohol of the formula:

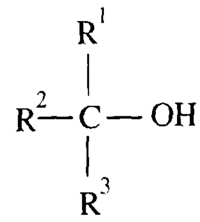

under acidic conditions as in the Ritter reaction.

A further method of preparing the compounds of the invention in which n is 1 comprises the desulphurisation of a sulphur containing compound of the formula:

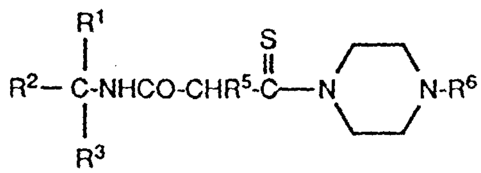

The desulphurisation may be carried out in the presence of a nickel catalyst. The sulphur containing compound may be prepared by a Willgerodt reaction, e.g. an aryl alkyl ketone of the formula $CH_3CO\text{-}R^5$ is reacted with sulphur and a piperazine of the formula:

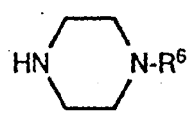

and the resulting thioamide is treated with a base and with an isocyanate of the formula:

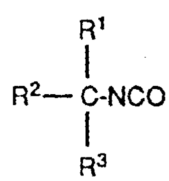

The processes described above may be carried out to give a compound of the invention in the form of a free base or as an acid addition salt. If the compound of the invention is obtained as an acid addition salt, the free base can be obtained by basifying a solution of the acid addition salt. Conversely, if the product of the process is a free base an acid addition salt, particularly a pharmaceutically acceptable acid addition salt, may be obtained by dissolving the free base in a suitable organic solvent and treating the solution with an acid, in accordance with conventional procedures for preparing acid addition salts from base compounds.

The following Examples illustrate, the preparation compounds of this invention.

## Example 1

**N-(1,1-Dimethylethyl)-4-(2-pyrimidinyl)-1-piperazinepropanamide**

To a suspension of t-butylamine hydrochloride (prepared by treatment of an $Et_2O$ solution of t-butylamine (2.7 mL, 1.9 g, 0.026 mol) with HCl) in a solution of 3-bromopropionic acid chloride (4.47 g, 0.0261 mol) with $CH_2Cl_2$ (145

mL) was added N,N-diisopropylethylamine (9.1 mL, 6.8 g, 0.052 mol) over 10-15 minutes at room temperature. The solution was stirred at room temperature for 2.5-3 hours. A few mL's of N,N-diisopropylethylamine were added until the solution became homogeneous. The solution was washed twice with dilute HCl and once with $H_2O$, dried with $MgSO_4$ and evaporated to provide 3.34 g of N-tert-butyl 3-bromopropanamide and N-tert-butyl acrylamide (approximately 36%/64% by weight). The reaction mixture was taken up in EtOH (200 mL), and to the solution of amides was added 1-(2-pyrimidinyl)piperazine dihydrochloride (6.82 g, 0.0288 mol) and sodium acetate (4.72 g, 0.0575 mol). The mixture was refluxed for 8 days. The solvent was evaporated. The residue was partitioned between $CH_2Cl_2$ and aqueous $Na_2CO_3$. The $CH_2Cl_2$ phase was washed with aqueous NaCl and dried with $MgSO_4$. Evaporation of the $CH_2Cl_2$ gave 5.61 of crude compound which was purified by HPLC to give 4.13 g of free base. Treatment in EtOH with HCl gave the title compound, 4.48 g (46%), mp 220-222°C.

| Elemental analysis for $C_{15}H_{25}N_5O \cdot 2HCl$ 1/2 $H_2O$ | | | |
|---|---|---|---|
| Calc'd | C, 48.25; | H, 7.55; | N, 18.76 |
| Found | C, 48.42; | H, 7.45; | N, 18.62 |

**Example 2**

**N-Tertiary-butyl 3-[4-(2-methoxyphenyl)piperazinyl]-2-phenyl-propanamide**

1-(2-Methoxyphenyl)piperazine (22.6 g, 0.118 mol) and atropic acid (17.4 g, 0.118 mol) in ethanol (300 mL) were heated under reflux for 18 hours, cooled to room temperature, and evaporated in vacuo. The solid was triturated with acetone (3 x 100 mL) to give a first crop of 3-[4-(2-methoxyphenyl)piperazinyl]-2-phenylpropionic acid (13.8 g) as white crystals. The filtrate was evaporated in vacuo to give an oil which slowly crystallized over 1 month. The solid was triturated with acetone (200 mL) to give a second crop of the same hemihydrate product (9.01 g) as white crystals, mp. 160-163°C.

| Elemental analysis for $C_{20}H_{24}N_2O_3 \cdot 1/2\ H_2O$ | | | |
|---|---|---|---|
| Calc'd | C, 68.8; | H, 7.2; | N, 8.0 |
| Found | C, 68.4; | H, 7.2; | N, 7.9 |

A stirred suspension of 3-[4-(2-methoxyphenyl)piperazinyl]-2-phenylpropionic acid (5.05 g; 14.7 mmol) in dichloromethane (30 ml) was treated with 1,1-carbonyldiimidazole (2.67 g, 16.5 mmol) and, after 35 minutes, the resulting solution was treated with tert-butylamine (1.9 ml, 18.2 mmol). After 18 hours, the mixture was evaporated in vacuo and the residue purified by chromatography (silica, diethyl ether) to give the product (2.80 g, 48%) as white crystals. A suspension of the solid in methanol-isopropanol (2 ml + 6 ml) was acidified with ethereal hydrogen chloride to give a solution which was evaporated in vacuo to give a solid. Trituration with diethyl ether gave the dihydrochloride salt of the product (3.30 g), mp. 230-231°C.

| Elemental analysis for $C_{24}H_{33}N_3O_2 \cdot 2HCl$ | | | |
|---|---|---|---|
| Calc'd | C, 61.5; | H, 7.5; | N, 9.0 |
| Found | C, 61.4; | H, 7.5; | N, 8.9 |

**Example 3**

**N-Tertiary-butyl 2-[[4-(2-methoxyphenyl)piperazinyl]methyl]-3-phenyl-propanamide**

2-(Phenylmethyl)propenoic acid (Mannich et al., Chem. Ber., 1922, 55, 3486) (2.00 g, 12.35 mmol) and 1-(2-methoxyphenyl)piperazine (2.37 g, 12.35 mmol) in propanol (25 mL) were boiled under reflux for 18 hours, cooled to room temperature, and evaporated in vacuo. The residue was triturated with acetone and diethyl ether to give 2-[[4-(2-methoxyphenyl)piperazinyl]methyl]-3-phenyl propanoic acid (0.80 g) as a colorless powder, mp. 155-158°C.

| Elemental analysis for $C_{21}H_{26}N_2O_3$ | | | |
|---|---|---|---|
| Calc'd | C, 71.2; | H, 7.3; | N, 7.9 |
| Found | C, 71.6; | H, 7.4; | N, 7.6 |

The product of the preceding paragraph (1.60 g, 4.5 mmol) in dichloromethane was treated with 1,1'-carbonyldiimidazole (0.73 g, 4.5 mmol), stirred for one hour, treated with tertiary butylamine (0.66 g, 9.0 mmol), stirred for eighteen hours, evaporated in vacuo and the residue was chromatographically purified (silica; diisopropyl ether gradient to diethyl ether). The product was dissolved in hot isopropanol and treated with ethereal HCl to obtain the title compound as the hydrochloride salt (0.43 g), mp. 127.5-130°C.

| Elemental analysis for $C_{25}H_{35}N_3O_2 \cdot HCl \cdot 3/4\ H_2O$ | | | |
|---|---|---|---|
| Calc'd | C, 65.4; | H, 8.2; | N, 9.2 |
| Found | C, 65.1; | H, 8.0; | N, 8.9 |

**Example 4**

**4-(2-Methoxyphenyl)-N-(1-methylcyclohexyl)-1-piperazinepropanamide**

To a suspension of l-methyl-cyclohexylamine hydrochloride (4.59 g, 0.0307 mol) in $CH_2Cl_2$ (150 mL) was added 3-bromopropionyl chloride (5.26 g, 0.0307 mol). Diisopropyl ethyl amine (20.7 mL, 15.4 g, 0.119 mol) was added slowly. The mixture was stirred at room temperature overnight. The organic phase was filtered, washed and aqueous HCl, water, and dried with $MgSO_4$. Evaporation of the $CH_2Cl_2$ gave 5.03 g of a mixture of N-1-methylcyclohexyl-2-bromopropanamide and N-1-methylcyclohexyl acrylamide in a ratio of 1 to 3. This mixture (3.0 g, 0.016 mol) was taken up in EtOH (142 mL). To this solution was added 1-(2-methoxyphenyl)piperazine hydrochloride (4.34 g, 0.0190 mol) and sodium acetate (1.58 g, 0.0193 mol). The mixture was refluxed two days. The EtOH was evaporated. The residue was partitioned between $H_2O$ and $CH_2Cl_2$. The $CH_2Cl_2$ phase dried with $MgSO_4$. Evaporation of $CH_2Cl_2$ gave crude product, 6.77 g. Purification by HPLC gave the pure free base, 2.38 g, which was treated with HCl in EtOH to give the title compound as the dihydrochloride, 2.53 g (37%, based on the amide mixture), mp. 198-203°C.

| Elemental analysis for $C_{21}H_{33}N_3O_2 \bullet 2HCl$ | | | |
|---|---|---|---|
| Calc'd | C, 58.33; | H, 8.16; | N, 9.72 |
| Found | C, 58.00; | H, 8.39, | N, 9.63 |

**Example 5**

**4-(2-Pyrimidinyl)-N-(1-methylcyclohexyl)-1-piperazinepropanamide**

The title compound was prepared from -1-(2-pyrimidyl)piperazine dihydrochloride (2.77 g, 0.0117 mol), sodium acetate (1.92 g, 0.0234 mol) and the mixture of 3-N-1-methylcyclohexyl-bromopropanamide and N-1-methylcyclohexyl acrylamide (1.8 g, 0.0098 mol) in the manner described in Example 5 to give 1.36 g of the title compound as the dihydrochloride (32%, based on the amide mixture), mp. 204-209°C.

| Elemental analysis for $C_{18}H_{29}N_5O \bullet 2\ HCl \bullet 3/2\ H_2O$ | | | |
|---|---|---|---|
| Calc'd | C, 50.1; | H, 7.94; | N, 16.24 |
| Found | C, 49.84; | H, 7.97; | N, 16.19 |

**Example 6**

**N-(1,1-Dimethylethyl)-3-[4-(2-methoxyphenyl)-1-piperazinyl]-propanamide**

A solution of N-tert-butylacrylamide (1.799 g, 14.2 mmol) and 1-(2-methoxyphenyl)piperazine (2.111 g, 11.0 mmol) in propanol (20 mL) was maintained at room temperture for 8 days, evaporated in vacuo, and chromatographed [silica; ethanolethyl acetate (1:20)] to give the free base of the product as a yellow oil (2.52 g, 72%).

The oil was dissolved in propan-2-ol (10 mL) and the solution acidified with ethereal hydrogen chloride. The mixture was evaporated in vacuo and the precipitate washed with diethyl ether to give the product as the dihydrochloride quarter hydrate (2.36 g, mp. 245-248°C (dec).

| Elemental analysis for $C_{18}H_{29}N_3O_2$•2HCl•1/4 $H_2O$ | | | |
|---|---|---|---|
| Calc'd | C, 54.5; | H, 8.0; | N, 10.6 |
| Found | C, 54.9; | H, 8.1; | N, 10.5 |

## Example 7

### N-Tert-butyl-2-methoxyphenyl-3-(4-(2-methoxyphenyl)-piperazin-1-yl)propanamide

A solution of potassium tert-butoxide (5.15 g, 46.0 mmols) in warm tert-butanol (21 ml) was added to a cooled (ice-methanol) suspension of 1-(2-[2-methoxyphenyl]-2-oxo)ethyl-4-(2-methoxyphenyl)piperazine dihydrochloride (5.00 gm, 12.11 mmols) and tosylmethylisocyanide (2.60 g, 13.31 mmols) in dry dimethoxyethane (100 mL). The reaction mixture was slowly allowed to warm to room temperature over 2 hours. Acetic acid (0.80 g, 13.33 mmols) was added to the reaction mixture followed by the addition of ethyl acetate (200 mL). The precipitate formed was filtered off and the filtrate concentrated to about 50 mL under reduced pressure. The concentrate was dissolved in water and washed with ethyl acetate. The combined organic phases were washed with brine, water, dried ($MgSO_4$) and concentrated to afford a brown oil. The crude product was chromatographed on silica gel and gradient eluted with hexane: ethyl acetate (3:2) to ethyl acetate, to afford a brown oil (3.30 g). A sample (1.0 g) of the oil was dissolved in diisopropylether and acidified with ethereal hydrogen chloride to afford off-white crystals which were recrystallized from diethylether in methanol to give 2-(2-methoxyphenyl)-3-(4-(2-methoxyphenyl)-piperazin-1-yl)propanenitrile as a dihydrochloride quarterhydrate as colourless crystals (0.75 g), mp. 189-192°C.

| Elemental analysis for $C_{21}H_{25}N_3O_2$·2HCl·1/4$H_2O$ | | | |
|---|---|---|---|
| Calc'd | C, 58.9; | H, 6.5; | N, 9.8 |
| Found | C, 58.7; | H, 6.5; | N, 9.5 |

Tert-butanol (1.20 g, 16.2 mmoles) was added to an ice-cooled solution of 2-(2-methoxyphenyl)-3-(4-(2-methoxyphenyl)piperazin-1yl)propanonitrile dihydrochloride (1.90 g, 5.41 mmoles) in methane-sulphonic acid (10.0 mL) and the reaction mixture stirred at room temperature for 48 hours. The reaction mixture was poured onto ice and basified with a mixture of 2M sodium hydroxide and solid sodium hydroxide. The aqueous layer was washed with ethyl acetate (3 x 30 mL) and the combined organic phases washed with brine (30 mL), water (30 mL), dried ($MgSO_4$) and concentrated to afford a brown oil. The crude product was chromatographed on silica gel and gradient eluted with ethyl acetate:hexane (1:1 to 4:1) and finally ethyl acetate to give a brown oil (2.06 g) which solidified on standing. The crude product was dissolved in diethyl ether and acidified with ethereal hydrogen chloride to afford a powder which was triturated in diisopropylether and then recrystallised from a mixture of diisopropylether and ethanol to afford the title compound as a dihydrochloride hydrate (0.30 g), mp. 129.0-132.5°C.

| Elemental analysis for $C_{25}H_{35}N_3O_3$•2HCl•$H_2O$ | | | |
|---|---|---|---|
| Calc'd | C, 58.1; | H, 7.6; | N, 8.1 |
| Found | C, 58.5; | H, 7.9; | N, 7.7 |

## Example 8

### (R*)- and (S*)-N-Tertiary butyl-3-[4-(2-methoxy)phenyl]piperazin-1-yl]-2-phenylpropionamide

A solution of di-p-toluoyl-L-tartaric acid monohydrate (4.67 g, 11.55 mmol) in acetonitrile (20 ml) was added in one portion to a solution of compound of Example 3, free base (4.57 g, 11.55 mmol) in acetonitrile (80 ml). The solution was concentrated in vacuo to give a white crystalline residue, which was redissolved in boiling acctonitrile (100 ml). The solution was allowed to cool overnight. The bulk solution was filtered, and the remaining crystals were collected, to give the first enantiomer as its di-p-toluoyl-L-tartrate (4.63 g). During the collection of the first crop of crystals, crystallisation was observed in the filtrate, and a second crop of crystals was collected separately, to give the second enantiomer as its di-p-toluoyl-L-tartrate (2.86 g). The remaining filtrate was concentrated in vacuo to give a foam (1.48 g). The di-p-toluoyl-L-tartrate of the first enantiomer was combined with the foam, and the mixture was recrystallised twice from the hot acetonitrile (100 ml) to give the (R*) title compound as its di-p-toluoyl-L-tartrate (3.64 g), m.p. 130°-145°C (dec.).

| Elemental analysis for $C_{24}H_{33}N_3O_2 \cdot C_{20}H_{18}O_8 \bullet 0.25\ H_2O$ | | | |
|---|---|---|---|
| Calc'd | C, 67.2; | H, 6.6; | N, 5.3 |
| Found | C, 67.0; | H, 6.8; | N, 5.3 |

The di-p-toluoyl-L-tartrate -tartrate of the second enantiomer was combined with the two residues of recrystallisation of the di-p-toluoyl-L-tartrate of the. first enantiomer, and the mixture was dissolved in chloroform (50 ml). The solution was washed with N aqueous sodium hydroxide (60 ml), and the aqueous phase was extracted with chloroform (50 ml). The combined chloroform phases were dried ($Na_2SO_4$), and concentrated in vacuo to give a white solid (2.50 g). The solid was dissolved in acetonitrile (25 ml), and a solution of di-p-toluoyl-L-tartaric acid (2.56 g, 6.32 mmol) in acetonitrile (5 ml) was added. The solution was concentrated in vacuo to give a solid residue, which was treated with acetonitrile (20 ml) and heated. During heating, crystallisation was observed, and the mixture was allowed to stand overnight. The product was collected to give the (S*)-title compound as its di-p-toluoyl-L-tartrate (4.08 g), 140°-145°C (dec.).

| Elemental analysis for $C_{24}H_{33}N_3O_2C_{20}H_{18}O_5 \bullet 0.25\ H_2O$ | | | |
|---|---|---|---|
| Calc'd | C, 67.2; | H, 6.6; | N, 5.3 |
| Found | C, 67.1; | H, 6.6; | N, 5.3 |

The di-p-toluoyl-L-tartrate -tartrate of the (R*)-title compound was dissolved in chloroform (50 ml), and the solution was washed with N aqueous sodium hydroxide (60 ml). The aqueous phase was extracted with chloroform (50 ml), and the combined chloroform phases were dried ($Na_2SO_4$), and concentrated in vacuo to give the (R*)-title compound as the free base, $[a]_D^{24}$ 7.4°C (c, 0.65 in $CHCl_3$). The free base was dissolved in ethyl acetate (90 ml) and the solution was acidified with ethereal hydrogen chloride (5 ml). The mixture was concentrated in vacuo, and the residue was triturated with hot acetonitrile (100 ml), to give the (R*)-title compound as the dihydrochloride (1.79 g, 76% overall) m.p. 200°-215°C (dec.), $[a]_D^{24}$ + 42°C (c, 1.1 in MeOH-$H_2O$, 1:7).

| Elemental analysis for $C_{24}H_{33}N_3O_2 \bullet 2\ HCl$ | | | |
|---|---|---|---|
| Calc'd | C, 61.5; | H, 7.5; | N, 9.0 |
| Found | C, 61.8; | H, 7.5; | N, 9.0 |

The di-p-toluoyl-L-tartrate of the (S*)-title compound was treated in the same way as the di-p-toluoyl-L-tartrate of the (R*)-title compound, to give the (S*)-title compound as the free base, $[a]_D^{24}$ + 8°C (c, 0.55 in $CHCl_3$). The free base was converted to its dihydrochloride with the same procedure used above to give the (S*)-title compound as the dihydrochloride (2.03 g, 86% overall), m.p. 200°-215°C (dec.), $[a]_D^{24}$ -44°C (c, 1.1 in MeOH-$H_2O$, 1:7).

| Elemental analysis for $C_{24}H_{33}N_3O_2 \bullet 2\ HCl$ | | | |
|---|---|---|---|
| Calc'd | C, 61.5; | H, 7.5; | N, 9.0 |
| Found | C, 61.3; | H, 7.3; | N, 8.9 |

The compounds of this invention have been demonstrated to possess $5HT_{1A}$ receptor binding properties which characterize them as useful anxiolytic and/or antidepressant agents. Where the compounds of this invention also display $D_2$ receptor site binding properties, they also have an anti-psychotic element which is of use in treatment of anxiety, depression, senile dementia (SDAT), Huntington's chorea, stroke and sexual disturbances.

Those compounds in which $R^5$ is alkyl of 4 to 8 carbon atoms or optionally substituted phenyl or benzyl, selectively bind to receptors of the 5-$HT_{1A}$ type to a much greater extent than they bind to other receptors such as $\alpha_1$ and $D_2$ receptors.

The pharmacological profile of the compounds of this invention resembles that of buspirone obtained by measuring the compound's ability to displace [$^3$H] 8-OH DPAT (dipropylaminotetralin) from the 5-$HT_{1A}$ serotonin receptor by the procedure of Hall et al., J. Neurochem. 44: 1685-1696, 1985. The compounds of this invention, like buspirone, exhibited potent affinity for this serotonin receptor subtype. The anxiolytic activity of buspirone is currently believed to be due, at least in part, to this receptor (Vander Maclen et al., Eur. J. Pharmacol. 1986, 129 (1-2) 123-130.

The $D_2$ dopamine receptor binding study was in accordance with a modification of the procedure of Field et al., Brain Res., 136, pp. 578-584 (1977) and Yamamura et al., eds., Neurotransmitter Receptor Binding, Raven Press,

N.Y. (1978), wherein homogenized limbic brain tissue is incubated with $^3$H-spiroperiodol and various concentrations of test compounds, filtered and washed and shaken with Hydrofluoro scintillation cocktail (National Diagnostics) and counted in a Packard 460CD scintillation counter. The test results of these studies are as follows:

| Compound of | Affinity for $5HT_{1A}$ Receptor % Inhibition | Affinity for $D_2$ Receptor % Inhibition |
|---|---|---|
| Example 2 | $IC_{50}$ 25.0 nM<br>86 at 1 µM<br>69 at 0.1 µM | $IC_{50}$ 1740 nM<br>32 at 1 µM<br>8 at 0.1 µM |
| Example 3 | $IC_{50}$ 11.4 nM<br>100 at 1 µM<br>90 at 0.1 µM | 38 at 10 µM |
| Compound of | Affinity for $5HT_{1A}$ Receptor % Inhibition | Affinity for $D_2$ Receptor % Inhibition |
| Example 4 | 56 at 0.1 µM | |
| Example 6 | $IC_{50}$ 287.0 nM<br>72 at 1 µM<br>18 at 0.1 µM | |

The very weak $D_2$ receptor binding exhibited by the compounds of Examples 2 and 3 illustrate the selective $5HT_{1A}$ receptor binding properties of those compounds mentioned, supra, where $R^5$ is alkyl of 4 to 8 carbon atoms or an optionally substituted phenyl or benzyl moiety.

Hence, the compounds of this invention are anxiolytic and/or antidepressant agents useful in the treatment of depression and in alleviating anxiety and in those instances where meaningful $D_2$ binding occurs as antipsychotic agents for treatment of psychoses such as paranoia and schizophrenia, as well as the related secondary conditions of sexual dysfunction, senile dementia (SDAT) and the like. As such, the compounds may be administered neat or with a pharmaceutical carrier to a patient in need thereof. The pharmaceutical carrier may be solid or liquid.

A solid carrier can include one or more substances which may also act as flavoring agents, lubricants, solubilizers, suspending agents, filters, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions. syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain suitable pharmaceutical additives such as solubilizers, emulsifiers, buffers, preservatives, sweeteners, flavoring agents, suspending agents, thickening agents, colors, viscosity regulators, stabilizers or osmoregulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycols) and their derivatives, and oils (e.g. fractionated coconut oil and arachis oil). For parenteral administration, the carrier can also be an oily ester such as ethyl oleate and isopropyl myristate. Sterile liquid carriers are used in sterile liquid form compositions for parenteral administration. The liquid carrier for pressurized compositions can be halogenated hydrocarbon or other pharmaceutically acceptable propellents.

Liquid pharmaceutical compositions which are sterile solutions or suspensions can be utilized by, for example, intramuscular, intraperitoneal or subcutaneous injection. Sterile solutions can also be administered intravenously. When the compound is orally active, it can be administered orally either in liquid or solid composition form.

Preferably, the pharmaceutical composition is in unit dosage form, e.g., as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate

number of any such compositions in package form.

The dosage to be used in the treatment of a specific psychosis must be subjectively determined by the attending physician. The variables involved include the specific psychosis of state of anxiety and the size, age and response of the patient.

## Claims

### Claims for the following Contracting States : AT, BE, CH, DE, DK, FR, IT, LI, LU, NL, SE

**1.** A compound of the formula:

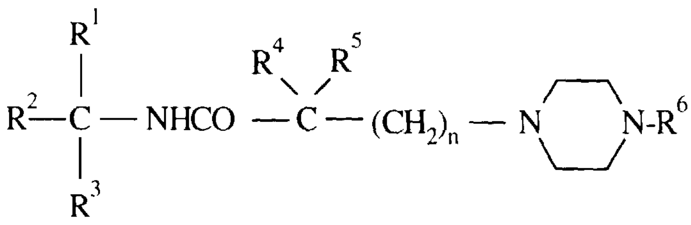

(I)

in which

| | |
|---|---|
| $R^1$ | is alkyl of 1 to 6 carbon atoms; |
| $R^2$ and $R^3$ | are alkyl of 1 to 6 carbon atoms or taken together they are polymethylene of 2 to 12 carbon atoms; |
| $R^4$ | is hydrogen or alkyl of 1 to 6 carbon atoms; |
| $R^5$ | is hydrogen, alkyl of 1 to 8 carbon atoms, hydroxyalkyl of 1 to 3 carbon atoms, phenyl, benzyl, substituted phenyl or substituted benzyl in which the substituents are hydroxy, halo, alkyl of 1 to 6 carbon atoms, alkoxy of 1 to 6 carbon atoms, trifluoromethyl, nitro, cyano, carbalkoxy of 2 to 7 carbon atoms, carboxamido, amino, alkylamino of 1 to 6 carbon atoms or dialkylamino of 2 to 12 carbon atoms; |
| $R^6$ | is phenyl, benzyl, 2-, 3-, or 4-pyridinyl, 2-pyrimidinyl or 2-pyrazinyl; any of which may be substituted by one or more hydroxy, halo, alkyl of 1 to 6 carbon atoms, alkoxy of 1 to 6 carbon atoms, trifluoromethyl, nitro, cyano, carbalkoxy of 2 to 7 carbon atoms, carboxamido, amino, alkylamino of 1 to 6 carbon atoms or dialkylamino of 2 to 12 carbon atoms; |
| and n | is one of the integers 1 or 2; or a pharmaceutically acceptable salt thereof. |

**2.** A compound as claimed in claim 1 in which

| | |
|---|---|
| $R^2$ and $R^3$ | are alkyl of 1 to 6 carbon atoms or taken together they are polymethylene of 2 to 6 carbon atoms; |
| $R^5$ | is hydrogen, alkyl of 1 to 6 carbon atoms, hydroxyalkyl of 1 to 3 carbon atoms, phenyl, benzyl, p-hydroxyphenyl, p-methoxyphenyl, o-methoxyphenyl, p-chlorophenyl or p-fluorophenyl; |
| and | |
| $R^6$ | is phenyl, 2-alkylphenyl in which the alkyl substituent has 1 to 6 carbon atoms, 2-alkoxyphenyl in which the alkoxy substituent has 1 to 6 carbon atoms, 2-halophenyl, 2-cyanophenyl, 2-nitrophenyl, 2-perhalomethylphenyl, benzyl, 2-, 3- or 4-pyridinyl, 2-pyrimidinyl or 2-pyrazinyl; |

or a pharmaceutically acceptable salt thereof.

3. A compound as claimed in Claim 1 in which $R^2$ and $R^3$ are, independently, alkyl of 1 to 6 carbon atoms.

4. A compound of Claim 1 in which $R^2$ and $R^3$ are tetramethylene, pentamethylene or hexamethylene.

5. A compound as claimed in any one of Claims 1, 3 or 4 in which $R^4$ is hydrogen and $R^5$ is alkyl of 1 to 6 carbon atoms, phenyl or benzyl.

6. A compound as claimed in any one of Claims 1, 2, 3, 4, or 5 in which $R^6$ is 2-methoxyphenyl, 2-chlorophenyl or 2-pyrimidinyl.

7. A compound as claimed in Claim 1 in which $R^5$ is alkyl or 4 to 8 carbon atoms, phenyl, benzyl, substituted phenyl or benzyl in which the substituents are halo, alkyl of 1 to 6 carbon atoms, alkoxy of 1 to 6 carbon atoms, trfluoromethyl, nitro, cyano, carbalkoxy of 2 to 7 carbon atoms, carboxamido, alkylamino of 1 to 6 carbon atoms or dialkylamino of 2 to 12 carbon atoms.

8. A compound as claimed in claim 7 in which n is 1 and $R^2$ and $R^3$ are, independently, alkyl of 1 to 6 carbon atoms.

9. The compound of Claim 1 which is

N-(1,1-dimethylethyl)-4-(2-pyrimidinyl)-1-piperazinepropanamide, or a pharmaceutically acceptable salt thereof, or

N-tertiary-butyl 3-[4-(2-methoxyphenyl)piperazinyl]-2-phenylpropanamide, or a pharmaceutically acceptable salt thereof, or

N-tertiary-butyl 2-[[4-(2-methoxyphenyl)piperazinyl]methyl]-3-phenylpropanamide, or a pharmaceutically acceptable salt thereof, or

4-(2-methoxyphenyl)-N-(1-methylcyclohexyl)-1-piperazinepropanamide, or a pharmaceutically acceptable salt thereof, or

4-(2-pyrimidinyl)-N-(1-methylcyclohexyl)-1-piperazinepropanamide, or a pharmaceutically acceptable salt thereof, or

(R)-1-methyl-N-[1-methyl-2-[4-(2-pyrimidinyl)]-1-piperazinyl]ethyl]cyclohexane carboxamide, or a pharmaceutically acceptable salt thereof, or

N-(1,1-dimethylethyl)-3-[4-(2-methoxyphenyl)-1-piperazinyl]-propanamide, or a pharmaceutically acceptable salt thereof, or

N-tert-butyl-2-methoxyphenyl-3-(4-(2-methoxypheny-piperazin-1-yl)propanamide, or a pharmaceutically acceptable salt thereof, or

(R*)-N-tertiary butyl-3-[4-(2-methoxy )phenyl]-piperazin-1-yl]-2-phenylpropionamide, or a pharmaceutically acceptable salt thereof, or

(S*)-N-tertiary butyl-3-[4-(2-methoxy)phenyl]-piperazin-1-yl]-2-phenylpropionamide, or a pharmaceutically acceptable salt thereof.

10. A process for preparing a compound claimed in Claim 1 which comprises:

(a) acylating an amine of formula

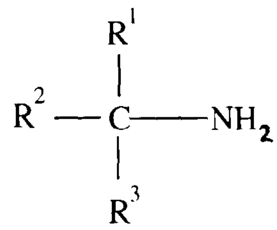

with an acid of formula

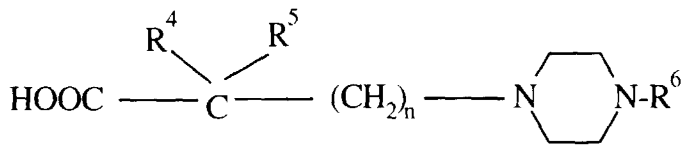

or with an acylating derivative thereof

(b) alkylating a piperazine of formula

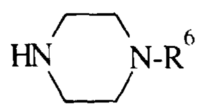

with an alkylating agent providing the group

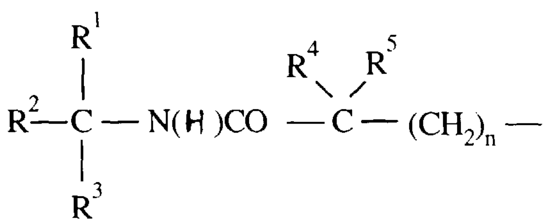

or

(c) converting a free base of formula (I) into a pharmaceutically acceptable salt thereof

or

(d) resolving a racemic compound of formula (I) to give an enantiomer.

**11.** A process for preparing a compound claimed in claim 1 which comprises reacting a nitrile of formula

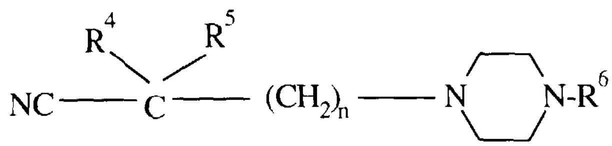

with a tertiary alcohol of formula

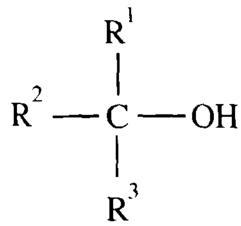

under acidic conditions.

**12.** A process for preparing a compound claimed in claim 1 in which $R^4$ is hydrogen and n is 1 which comprises desulphurising a sulphur containing compound of formula

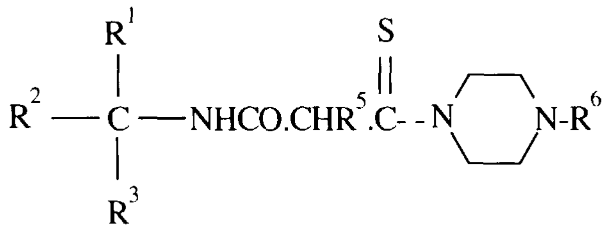

**13.** A pharmaceutical composition comprising a compound claimed in any one of claims 1 to 9 in association with a pharmaceutically acceptable carrier.

**14.** A compound as described in any one of claims 1 to 9 for use as a pharmaceutical.

**Claims for the following Contracting States : ES, GR**

**1.** A process for preparing a compound of the formula:

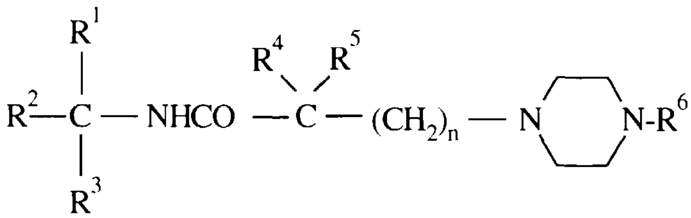

(I)

in which

$R^1$ is alkyl of 1 to 6 carbon atoms;

$R^2$ and $R^3$ are alkyl of 1 to 6 carbon atoms or taken together they are polymethylene of 2 to 12 carbon atoms;

$R^4$ is hydrogen or alkyl of 1 to 6 carbon atoms;

$R^5$ is hydrogen, alkyl of 1 to 8 carbon atoms, hydroxyalkyl of 1 to 3 carbon atoms, phenyl, benzyl, substituted phenyl or substituted benzyl in which the substituents are hydroxy, halo, alkyl of 1 to 6 carbon atoms, alkoxy of 1 to 6 carbon atoms, trifluoromethyl, nitro, cyano, carbalkoxy of 2 to 7 carbon atoms, carboxamido, amino, alkylamino of 1 to 6 carbon atoms or dialkylamino of 2 to 12 carbon atoms;

$R^6$ is phenyl, benzyl, 2-, 3-, or 4-pyridinyl, 2-pyrimidinyl or 2-pyrazinyl; any of which may be substituted by one or more hydroxy, halo, alkyl of 1 to 6 carbon atoms, alkoxy of 1 to 6 carbon atoms, trifluoromethyl, nitro, cyano, carbalkoxy of 2 to 7 carbon atoms, carboxamido, amino. alkylamino of 1 to 6 carbon atoms or dialkylamino of 2 to 12 carbon atoms;

and n is one of the integers 1 or 2; or a pharmaceutically acceptable salt thereof,

which comprises

(a) acylating an amine of formula

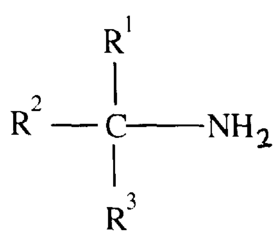

with an acid of formula

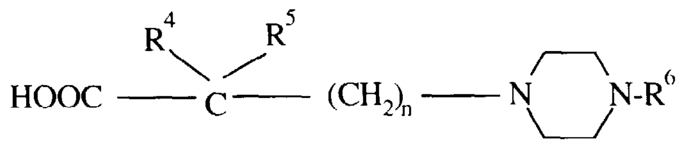

or with an acylating derivative thereof

(b) alkylating a piperazine of formula

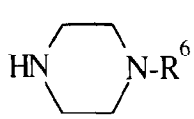

with an alkylating agent providing the group

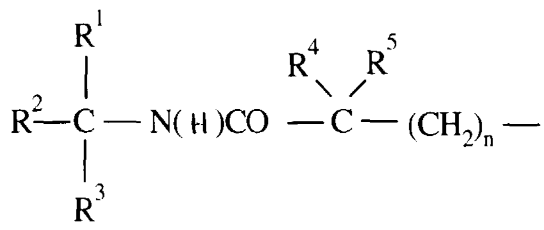

or

(c) reacting a nitrile of formula

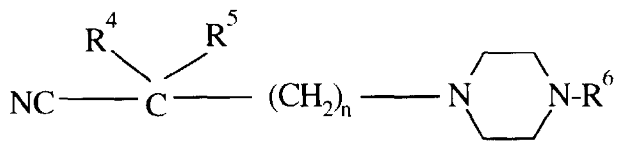

with a tertiary alcohol of formula

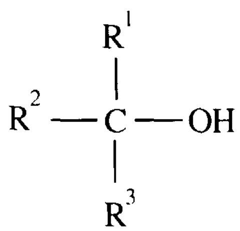

under acidic conditions

or

(d) desulphurising a sulphur containing compound of formula

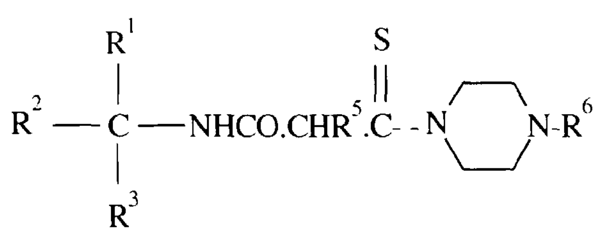

to give a compound of formula I in which $R^4$ is hydrogen and n is 1 or

(e) converting a free base of formula (I) into a pharmaceutically acceptable salt thereof

or

(f) resolving a racemic compound of formula (I) to give an enantiomer.

**2.** A process as claimed in claim 1 in which, in the product,

$R^2$ and $R^3$ are alkyl of 1 to 6 carbon atoms or taken together they are polymethylene of 2 to 6 carbon atoms;

$R^5$ is hydrogen, alkyl of 1 to 6 carbon atoms, hydroxyalkyl of 1 to 3 carbon atoms, phenyl, benzyl, p-hydroxyphenyl, p-methoxyphenyl, o-methoxyphenyl, p-chlorophenyl or p-fluorophenyl;

and

$R^6$ is phenyl, 2-alkylphenyl in which the alkyl substituent has 1 to 6 carbon atoms, 2-alkoxyphenyl in which the alkoxy substituent has 1 to 6 carbon atoms, 2-halophenyl, 2-cyanophenyl, 2-nitrophenyl, 2-perhalomethylphenyl, benzyl, 2-, 3- or 4-pyridinyl, 2-pyrimidinyl or 2-pyrazinyl;

or a pharmaceutically acceptable salt thereof.

**3.** A process as claimed in Claim 1 in which, in the product, $R^2$ and $R^3$ are, independently, alkyl of 1 to 6 carbon atoms.

**4.** A process as claimed in Claim 1 in which, in the product, $R^2$ and $R^3$ are tetramethylene, pentamethylene or hexamethylene.

**5.** A process as claimed in any one of Claims 1, 3 or 4 in which, in the product, $R^4$ is hydrogen and $R^5$ is alkyl of 1 to 6 carbon atoms, phenyl or benzyl.

**6.** A process as claimed in any one of Claims 1, 2, 3, 4, or 5 in which, in the product, $R^6$ is 2-methoxyphenyl, 2-chlorophenyl or 2-pyrimidinyl.

**7.** A process as claimed in Claim 1 in which, in the product, $R^5$ is alkyl or 4 to 8 carbon atoms, phenyl, benzyl, substituted phenyl or benzyl in which the substituents are halo, alkyl of 1 to 6 carbon atoms, alkoxy of 1 to 6 carbon atoms, trfluoromethyl, nitro, cyano, carbalkoxy of 2 to 7 carbon atoms, carboxamido, alkylamino of 1 to 6 carbon atoms or dialkylamino of 2 to 12 carbon atoms.

**8.** A process as claimed in claim 7 in which, in the product, n is 1 and $R^2$ and $R^3$ are, independently, alkyl of 1 to 6 carbon atoms.

**9.** The process as claimed in Claim 1 in which the product is

N-(1,1-dimethylethyl)-4-(2-pyrimidinyl)-1-piperazinepropanamide, or a pharmaceutically acceptable salt thereof, or

N-tertiary-butyl 3-[4-(2-methoxyphenyl)piperazinyl]-2-phenylpropanamide, or a pharmaceutically acceptable salt thereof, or

N-tertiary-butyl 2-[[4-(2-methoxyphenyl)piperazinyl]methyl]-3-phenylpropanamide, or a pharmaceutically acceptable salt thereof, or

4-(2-methoxyphenyl)-N-( 1 -methylcyclohexyl)-1-piperazinepropanamide, or a pharmaceutically acceptable salt thereof, or

4-(2-pyrimidinyl)-N-( 1 -methylcyclohexyl)-1-piperazinepropanamide, or a pharmaceutically acceptable salt thereof, or

(R)-1-methyl-N-[1-methyl-2-[4-(2-pyrimidinyl)]-1-piperazinyl]ethyl]cyclohexane carboxamide, or a pharmaceutically acceptable salt thereof, or

N-(1,1-dimethylethyl)-3-[4-(2-methoxyphenyl)-1-piperazinyl]-propanamide, or a pharmaceutically acceptable salt thereof, or

N-tert-butyl-2-methoxyphenyl-3-(4-(2-methoxypheny-piperazin-1-yl)propanamide, or a pharmaceutically acceptable salt thereof, or

(R*)-N-tertiary butyl-3-[4-(2-methoxy)phenyl]-piperazin-1-yl]-2-phenylpropionamide, or a pharmaceutically acceptable salt thereof, or

(S*)-N-tertiary butyl-3-[4-(2-methoxy)phenyl]-piperazin-1-yl]-2-phenylpropionamide, or a pharmaceutically acceptable salt thereof.

**10.** A process for preparing a pharmaceutical composition comprising bringing a compound of formula (I), as defined in claim 1, or a pharmaceutically acceptable salt thereof, into association with a pharmaceutically acceptable carrier.

**11.** A process as claimed in claim 10 wherein the active ingredient is prepared by the process claimed in any one of claims 1 to 9.

**Patentansprüche**

**Patentansprüche für folgende Vertragsstaaten : AT, BE, CH, DE, DK, FR, IT, LI, LU, NL, SE**

**1.** Verbindung der Formel:

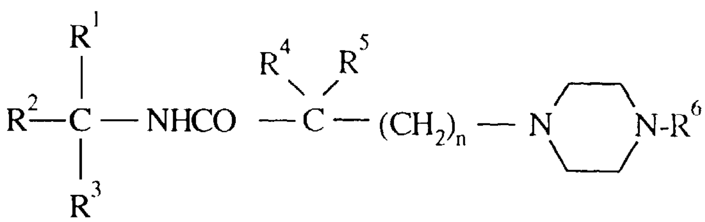

(I)

in welcher

$R^1$ Alkyl mit 1 bis 6 Kohlenstoffatomen darstellt;
$R^2$ und $R^3$ Alkyl mit 1 bis 6 Kohlenstoffatomen darstellen oder zusammengenommen Polymethylen mit 2 bis 12 Kohlenstoffatomen darstellen;
$R^4$ Wasserstoff oder Alkyl mit 1 bis 6 Kohlenstoffatomen darstellt;
$R^5$ Wasserstoff, Alkyl mit 1 bis 8 Kohlenstoffatomen, Hydroxyalkyl mit 1 bis 3 Kohlenstoffatomen, Phenyl, Benzyl, substituiertes Phenyl oder substituiertes Benzyl, in welchem die Substituenten sind: Hydroxy, Halogen, Alkyl mit 1 bis 6 Kohlenstoffatomen, Alkoxy mit 1 bis 6 Kohlenstoffatomen, Trifluormethyl, Nitro, Cyano, Carbalkoxy mit 2 bis 7 Kohlenstoffatomen, Carboxamido, Amino, Alkylamino mit 1 bis 6 Kohlenstoffatomen oder Dialkylamino mit 2 bis 12 Kohlenstoffatomen darstellt;
$R^6$ Phenyl, Benzyl, 2-, 3- oder 4-Pyridinyl, 2-Pyrimidinyl oder 2-Pyrazinyl; von denen jedes substituiert sein kann mit einem oder mehr Hydroxy, Halogen, Alkyl mit 1 bis 6 Kohlenstoffatomen, Alkoxy mit 1 bis 6 Kohlenstoffatomen, Trifluormethyl, Nitro, Cyano, Carbalkoxy mit 2 bis 7 Kohlenstoffatomen, Carboxamido, Amino, Alkylamino mit 1 bis 6 Kohlenstoffatomen oder Dialkylamino mit 2 bis 12 Kohlenstoffatomen darstellt;
und n eine der ganzen Zahlen 1 oder 2 ist; oder ein pharmazeutisch annehmbares Salz davon.

**2.** Verbindung wie in Anspruch 1 beansprucht, in welcher

$R^2$ und $R^3$ Alkyl mit 1 bis 6 Kohlenstoffatomen darstellen oder zusammengenommen Polymethylen mit 2 bis 6 Kohlenstoffatomen darstellen;
$R^5$ Wasserstoff, Alkyl mit 1 bis 6 Kohlenstoffatomen, Hydroxyalkyl mit 1 bis 3 Kohlenstoffatomen, Phenyl, Benzyl, p-Hydroxyphenyl, p-Methoxyphenyl, o-Methoxyphenyl, p-Chlorphenyl oder p-Fluorphenyl darstellt; und
$R^6$ Phenyl,2-Alkylphenyl, in welchem der Alkyl-Substituent 1 bis 6 Kohlenstoffatome aufweist, 2-Alkoxyphenyl, in welchem der Alkoxy-Substituent 1 bis 6 Kohlenstoffatome aufweist, 2-Halogenphenyl, 2-Cyanophenyl, 2-Nitrophenyl, 2-Perhalogenmethylphenyl, Benzyl, 2-, 3- oder 4-Pyridinyl, 2-Pyrimidinyl oder 2-Pyrazinyl darstellt;

oder ein pharmazeutisch annehmbares Salz davon.

3. Verbindung wie in Anspruch 1 beansprucht, in welcher $R^2$ und $R^3$ unabhängig Alkyl mit 1 bis 6 Kohlenstoffatomen darstellen.

4. Verbindung von Anspruch 1, in welcher $R^2$ und $R^3$ Tetramethylen, Pentamethylen oder Hexamethylen darstellen.

5. Verbindung wie in einem der Ansprüche 1, 3 oder 4 beansprucht, in welcher $R^4$ Wasserstoff darstellt und $R^5$ Alkyl mit 1 bis 6 Kohlenstoffatomen, Phenyl oder Benzyl darstellt.

6. Verbindung wie in einem der Ansprüche 1, 2, 3, 4 oder 5 beansprucht, in welcher $R^6$ 2-Methoxyphenyl, 2-Chlorphenyl oder 2-Pyrimidinyl darstellt.

7. Verbindung wie in Anspruch 1 beansprucht, in welcher $R^5$ Alkyl mit 4 bis 8 Kohlenstoffatomen, Phenyl, Benzyl, substituiertes Phenyl oder Benzyl, in welchen die Substituenten sind: Halogen, Alkyl mit 1 bis 6 Kohlenstoffatomen, Alkoxy mit 1 bis 6 Kohlenstoffatomen, Trifluormethyl, Nitro, Cyano, Carbalkoxy mit 2 bis 7 Kohlenstoffatomen, Carboxamido, Alkylamino mit 1 bis 6 Kohlenstoffatomen oder Dialkylamino mit 2 bis 12 Kohlenstoffatomen darstellt.

8. Verbindung wie in Anspruch 7 beansprucht, in welcher n 1 ist und $R^2$ und $R^3$ unabhängig Alkyl mit 1 bis 6 Kohlenstoffatomen darstellen.

9. Verbindung von Anspruch 1, welche ist:

N-(1,1-Dimethylethyl)-4-(2-pyrimidinyl)-1-piperazinpropanamid oder ein pharmazeutisch annehmbares Salz davon, oder
N-tertiäres Butyl-3-[4-(2-methoxyphenyl)piperazinyl]-2-phenylpropanamid oder ein pharmazeutisch annehmbares Salz davon, oder
N-tertiäres Butyl-2-[[4-(2-methoxyphenyl)piperazinyl]methyl]-3-phenylpropanamid oder ein pharmazeutisch annehmbares Salz davon, oder
4-(2-Methoxyphenyl)-N-(1-methylcyclohexyl)-1-piperazinpropanamid oder ein pharmazeutisch annehmbares Salz davon, oder
4-(2-Pyrimidinyl)-N-(1-methylcyclohexyl)-1-piperazinpropanamid oder ein pharmazeutisch annehmbares Salz davon, oder
(R)-1-Methyl-N-[1-methyl-2-[4-(2-pyrimidinyl)]-1-piperazinyl]ethyl]cyclohexancarboxamid oder ein pharmazeutisch annehmbares Salz davon, oder
N-(1,1-Dimethylethyl)-3-[4-(2-methoxyphenyl)-1-piperazinyl]-propanamid oder ein pharmazeutisch annehmbares Salz davon, oder
N-tert-Butyl-2-methoxyphenyl-3-(4-(2-methoxyphenyl-piperazin-1-yl)propanamid oder ein pharmazeutisch annehmbares Salz davon, oder
(R*)-N-tertiäres Butyl-3-[4-(2-methoxy)phenyl]-piperazin-1-yl]-2-phenylpropionamid oder ein pharmazeutisch annehmbares Salz davon, oder
(S*)-N-tertiäres Butyl-3-[4-(2-methoxy)phenyl]-piperazin-1-yl]-2-phenylpropionamid oder ein pharmazeutisch annehmbares Salz davon.

10. Verfahren zum Herstellen einer in Anspruch 1 beanspruchten Verbindung, welches umfaßt:

(a) Acylieren eines Amin der Formel

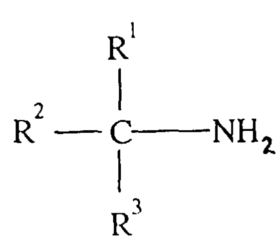

mit einer Säure der Formel

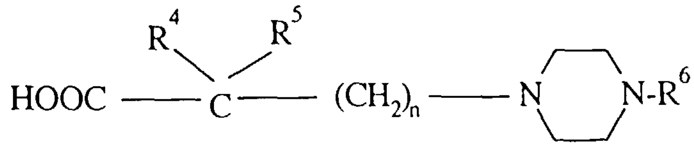

oder mit einem acylierenden Derivat davon
(b) Alkylieren eines Piperazin der Formel

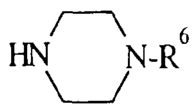

mit einem Alkylierungsmittel, die Gruppe

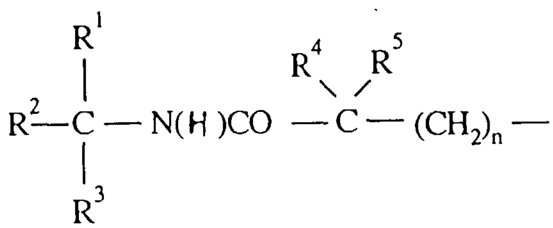

vorsehend, oder
(c) Umwandeln einer freien Base der Formel (I) in ein pharmazeutisch annehmbares Salz davon oder
(d) Aufspalten einer racemischen Verbindung der Formel (I), um ein Enantiomer zu ergeben.

**11.** Verfahren zum Herstellen einer in Anspruch 1 beanspruchten Verbindung, welches umfaßt:

Umsetzen eines Nitril der Formel

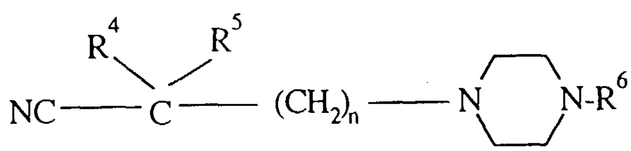

mit einem tertiären Alkohol der Formel

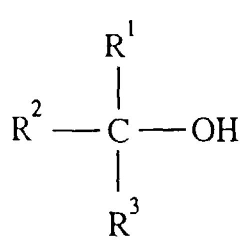

unter sauren Bedingungen.

**12.** Verfahren zum Herstellen einer in Anspruch 1 beanspruchten Verbindung, in welcher $R^4$ Wasserstoff darstellt und

n 1 ist, welches umfaßt:

Entschwefeln einer schwefelhältigen Verbindung der Formel

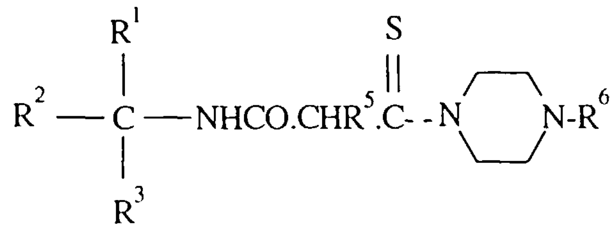

**13.** Pharmazeutische Zusammensetzung, umfassen eine in einem der Ansprüche 1 bis 9 beanspruchten Verbindung in Assoziation mit einem pharmazeutisch annehmbaren Träger.

**14.** Verbindung wie in einem der Ansprüche 1 bis 9 beschrieben, zum Verwenden als Pharmazeutikum.

**Patentansprüche für folgende Vertragsstaaten : ES, GR**

**1.** Verfahren zum Herstellen einer Verbindung der Formel

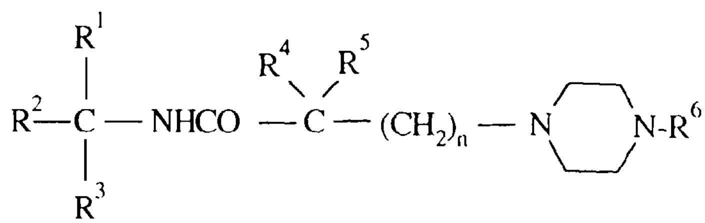

(I)

in welcher

$R^1$ Alkyl mit 1 bis 6 Kohlenstoffatomen darstellt;
$R^2$ und $R^3$ Alkyl mit 1 bis 6 Kohlenstoffatomen darstellen oder zusammengenommen Polymethylen mit 2 bis 12 Kohlenstoffatomen darstellen;
$R^4$ Wasserstoff oder Alkyl mit 1 bis 6 Kohlenstoffatomen darstellt;
$R^5$ Wasserstoff, Alkyl mit 1 bis 8 Kohlenstoffatomen, Hydroxyalkyl mit 1 bis 3 Kohlenstoffatomen, Phenyl, Benzyl, substituiertes Phenyl oder substituiertes Benzyl, in welchem die Substituenten sind: Hydroxy, Halogen, Alkyl mit 1 bis 6 Kohlenstoffatomen, Alkoxy mit 1 bis 6 Kohlenstoffatomen, Trifluormethyl, Nitro, Cyano, Carbalkoxy mit 2 bis 7 Kohlenstoffatomen, Carboxamido, Amino, Alkylamino mit 1 bis 6 Kohlenstoffatomen oder Dialkylamino mit 2 bis 12 Kohlenstoffatomen darstellt;
$R^6$ Phenyl, Benzyl, 2-, 3- oder 4-Pyridinyl, 2-Pyrimidinyl oder 2-Pyrazinyl; von denen jedes substituiert sein kann mit einem oder mehr Hydroxy, Halogen, Alkyl mit 1 bis 6 Kohlenstoffatomen, Alkoxy mit 1 bis 6 Kohlenstoffatomen, Trifluormethyl, Nitro, Cyano, Carbalkoxy mit 2 bis 7 Kohlenstoffatomen, Carboxamido, Amino, Alkylamino mit 1 bis 6 Kohlenstoffatomen oder Dialkylamino mit 2 bis 12 Kohlenstoffatomen darstellt;
und n eine der ganzen Zahlen 1 oder 2 ist; oder ein pharmazeutisch annehmbares Salz davon,

welches umfaßt:

(a) Acylieren eines Amin der Formel

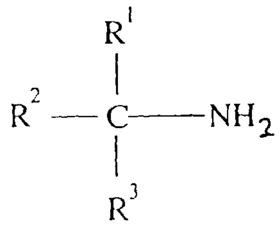

mit einer Säure der Formel

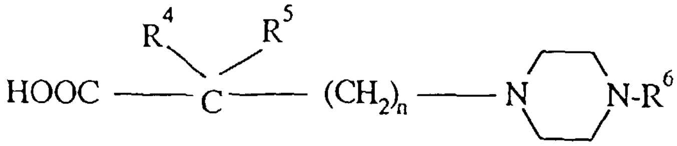

oder mit einem acylierenden Derivat davon
(b) Alkylieren eines Piperazin der Formel

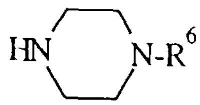

mit einem Alkylierungsmittel welches die Gruppe

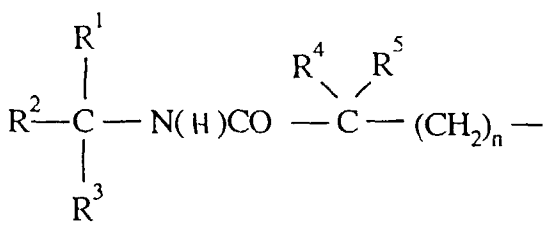

vorsieht, oder
(c) Umsetzen eines Nitril der Formel

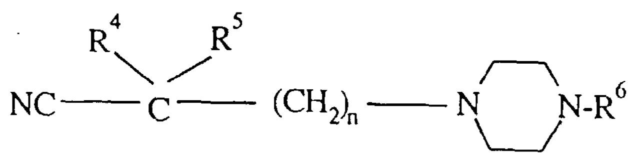

mit einem tertiären Alkohol der Formel

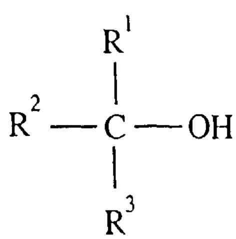

unter sauren Bedingungen oder

(d) Entschwefeln einer schwefelhältigen Verbindung der Formel

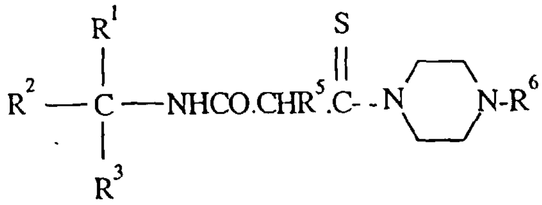

um eine Verbindung der Formel 1 zu ergeben, in welcher $R^4$ Wasserstoff darstellt und n 1 ist oder

(e) Umwandeln einer freien Base der Formel (I) in ein pharmazeutisch annehmbares Salz davon oder

(f) Aufspalten einer racemischen Verbindung der Formel (I), um ein Enantiomer zu ergeben.

**2.** Verfahren wie in Anspruch 1 beansprucht, wobei in dem Produkt

$R^2$ und $R^3$ Alkyl mit 1 bis 6 Kohlenstoffatomen darstellen oder zusammengenommen Polymethylen mit 2 bis 6 Kohlenstoffatomen darstellen;

$R^5$ Wasserstoff, Alkyl mit 1 bis 6 Kohlenstoffatomen, Hydroxyalkyl mit 1 bis 3 Kohlenstoffatomen, Phenyl, Benzyl, p-Hydroxyphenyl, p-Methoxyphenyl, o-Methoxyphenyl, p-Chlorphenyl oder p-Fluorphenyl darstellt; und

$R^6$ Phenyl, 2-Alkylphenyl, in welchem der Alkyl-Substituent 1 bis 6 Kohlenstoffatome aufweist, 2-Alkoxyphenyl, in welchem der Alkoxy-Substituent 1 bis 6 Kohlenstoffatome aufweist, 2-Halogenphenyl, 2-Cyanophenyl, 2-Nitrophenyl, 2-Perhalogenmethylphenyl, Benzyl, 2-, 3- oder 4-Pyridinyl, 2-Pyrimidinyl oder 2-Pyrazinyl darstellt;

oder ein pharmazeutisch annehmbares Salz davon.

**3.** Verfahren wie in Anspruch 1 beansprucht, bei welchem in dem Produkt $R^2$ und $R^3$ unabhängig Alkyl mit 1 bis 6 Kohlenstoffatomen darstellen.

**4.** Verfahren wie in Anspruch 1 beansprucht, bei welchem in dem Produkt $R^2$ und $R^3$ Tetramethylen, Pentamethylen oder Hexamethylen darstellen.

**5.** Verfahren wie in einem der Ansprüche 1, 3 oder 4 beansprucht, bei welchem in dem Produkt $R^4$ Wasserstoff darstellt und $R^5$ Alkyl mit 1 bis 6 Kohlenstoffatomen, Phenyl oder Benzyl darstellt.

**6.** Verfahren wie in einem der Ansprüche 1, 2, 3, 4 oder 5 beansprucht, bei welchem in dem Produkt $R^6$ 2-Methoxyphenyl, 2-Chlorphenyl oder 2-Pyrimidinyl darstellt.

**7.** Verfahren wie in Anspruch 1 beansprucht, bei welchem in dem Produkt $R^5$ Alkyl mit 4 bis 8 Kohlenstoffatomen, Phenyl, Benzyl, substituiertes Phenyl oder Benzyl, in welchen die Substituenten sind: Halogen, Alkyl mit 1 bis 6 Kohlenstoffatomen, Alkoxy mit 1 bis 6 Kohlenstoffatomen, Trifluormethyl, Nitro, Cyano, Carbalkoxy mit 2 bis 7 Kohlenstoffatomen, Carboxamido, Alkylamino mit 1 bis 6 Kohlenstoffatomen oder Dialkylamino mit 2 bis 12 Kohlenstoffatomen darstellt.

**8.** Verfahren wie in Anspruch 7 beansprucht, bei welchem in dem Produkt n 1 ist und $R^2$ una $R^3$ unabnängig Alkyl mit 1 bis 6 Kohlenstoffatomen darstellen.

**9.** Verfahren wie in Anspruch 1 beansprucht, bei welchem das Produkt

N-(1,1-Dimethylethyl)-4-(2-pyrimidinyl)-1-piperazinpropanamid oder ein pharmazeutisch annehmbares Salz davon, oder

N-tertiäres Butyl-3-[4-(2-methoxyphenyl)piperazinyl]-2-phenylpropanamid oder ein pharmazeutisch annehmbares Salz davon, oder

N-tertiäres Butyl-2-[[4-(2-methoxyphenyl)piperazinyl]methyl]-3-phenylpropanamid oder ein pharmazeutisch annehmbares Salz davon, oder

4-(2-Methoxyphenyl)-N-(1-methylcyclohexyl)-1-piperazinpropanamid oder ein pharmazeutisch annehmbares

Salz davon, oder
4-(2-Pyrimidinyl)-N-(1-methylcyclohexyl)-1-piperazinpropanamid oder ein pharmazeutisch annehmbares Salz davon, oder
(R)-1-Methyl-N-[1-methyl-2-[4-(2-pyrimidinyl)]-1-piperazinyl]ethyl]cyclohexancarboxamid oder ein pharmazeutisch annehmbares Salz davon, oder
N-(1,1-Dimethylethyl)-3-[4-(2-methoxyphenyl)-1-piperazinyl]-propanamid oder ein pharmazeutisch annehmbares Salz davon, oder
N-tert-Butyl-2-methoxyphenyl-3-(4-(2-methoxyphenyl-piperazin-1-yl)propanamid oder ein pharmazeutisch annehmbares Salz davon, oder
(R*)-N-tertiäres Butyl-3-[4-(2-methoxy)phenyl]-piperazin-1-yl]-2-phenylpropionamid oder ein pharmazeutisch annehmbares Salz davon, oder
(S*)-N-tertiäres Butyl-3-[4-(2-methoxy)phenyl]-piperazin-1-yl]-2-phenylpropionamid oder ein pharmazeutisch annehmbares Salz davon.

**10.** Verfahren zum Herstellen einer pharmazeutischen Zusammensetzung, welches das in Assoziation bringen einer Verbindung der Formel (I), wie in Anspruch 1 definiert, oder eines pharmazeutisch annehmbares Salzes davon mit einem pharmazeutisch annehmbaren Träger umfasst.

**11.** Verfahren wie in Anspruch 10 beansprucht, wobei der Wirkstoff durch das in einem der Ansprüche 1 bis 9 beanspruchte Verfahren hergestellt wird.

**Revendications**

**Revendications pour les Etats contractants suivants : AT, BE, CH, DE, DK, FR, IT, LI, LU, NL, SE**

**1.** Composé de formule

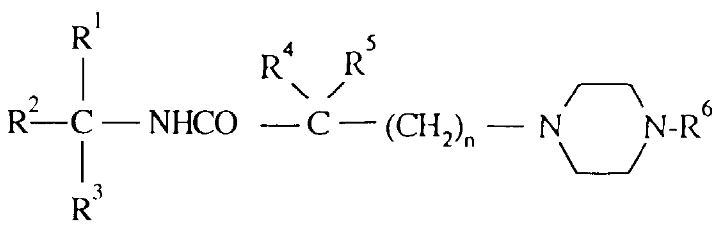

(I)

dans laquelle

$R^1$ est un alkyle de 1 à 6 atomes de carbone ;
$R^2$ et $R^3$ sont des alkyles de 1 à 6 atomes de carbone ou, pris conjointement, forment un polyméthylène de 2 à 12 atomes de carbone ;
$R^4$ est un hydrogène ou un alkyle de 1 à 6 atomes de carbone ;
$R^5$ est un hydrogène, alkyle de 1 à 8 atomes de carbone, hydroxyalkyle de 1 à 3 atomes de carbone, phényle, benzyle, phényle substitué ou benzyle substitué dans lequel les substituants sont hydroxy, halogène, alkyle de 1 à 6 atomes de carbone, alkoxy de 1 à 6 atomes de carbone, trifluorométhyle, nitro, cyano, carbalkoxy de 2 à 7 atomes de carbone, carboxamido, amino, alkylamino de 1 à 6 atomes de carbone ou dialkylamino de 2 à 12 atomes de carbone ;
$R^6$ est un phényle, benzyle, 2-, 3- ou 4-pyridinyle, 2-pyrimidinyle ou 2-pyrazinyle ; tous pouvant être substitués par un ou plusieurs hydroxy, halogène, alkyle de 1 à 6 atomes de carbone, alkoxy de 1 à 6 atomes de carbone, trifluorométhyle, nitro, cyano, carbalkoxy de 2 à 7 atomes de carbone, carboxamido, amino, alkylamino de 1 à 6 atomes de carbone ou dialkylamino de 2 à 12 atomes de carbone ;
et n est un des entiers 1 ou 2 ; ou un sel pharmaceutiquement acceptable de celui-ci.

**2.** Composé selon la revendication 1 dans lequel

$R^2$ et $R^3$ sont des alkyles de 1 à 6 atomes de carbone ou, pris conjointement, forment un polyméthylène de 2 à 6 atomes de carbone ;
$R^5$ est un hydrogène, alkyle de 1 à 6 atomes de carbone, hydroxyalkyle de 1 à 3 atomes de carbone, phényle, benzyle, p-hydroxyphényle, p-méthoxyphényle, o-méthoxyphényle, p-chlorophényle ou p-fluorophényle ;

et

$R^6$ est un phényle, 2-alkylphényle dans lequel le substituant alkyle a 1 à 6 atomes de carbone, 2-alkoxyphényle dans lequel le substituant alkoxy a 1 à 6 atomes de carbone, 2-halophényle, 2-cyanophényle, 2-nitrophényle, 2-perhalométhylphényle, benzyle, 2-, 3-ou 4-pyridinyle, 2-pyrimidinyle ou 2-pyrazinyle ;

ou un sel pharmaceutiquement acceptable de celui-ci.

**3.** Composé selon la revendication 1 dans lequel $R^2$ et $R^3$ sont, indépendamment, des alkyles de 1 à 6 atomes de carbone.

**4.** Composé selon la revendication 1 dans lequel $R^2$ et $R^3$ forment le tétraméthylène, le pentaméthylène ou l'hexaméthylène.

**5.** Composé selon l'une quelconque des revendications 1, 3 ou 4 dans lequel $R^4$ est un hydrogène et $R^5$ est un alkyle de 1 à 6 atomes de carbone, un phényle ou un benzyle.

**6.** Composé selon l'une quelconque des revendications 1, 2, 3, 4 ou 5 dans lequel $R^6$ est le 2-méthoxyphényle, le 2-chlorophényle ou le 2-pyrimidinyle.

**7.** Composé selon la revendication 1 dans lequel $R^5$ est un alkyle de 4 à 8 atomes de carbone, phényle, benzyle, phényle substitué ou benzyle substitué dans lequel les substituants sont halogène, alkyle de 1 à 6 atomes de carbone, alkoxy de 1 à 6 atomes de carbone, trifluorométhyle, nitro, cyano, carbalkoxy de 2 à 7 atomes de carbone, carboxamido, alkylamino de 1 à 6 atomes de carbone ou dialkylamino de 2 à 12 atomes de carbone.

**8.** Composé selon la revendication 7 dans lequel n est 1 et $R^2$ et $R^3$ sont indépendamment, des alkyles de 1 à 6 atomes de carbone.

**9.** Composé selon la revendication 1 qui est

le N-(1,1-diméthyléthyl)-4-(2-pyrimidinyl)-1-pipérazine-propanamide, ou un sel pharmaceutiquement acceptable de celui-ci, ou
le N-t-butyl-3-[4-(2-méthoxyphényl)pipérazinyl]-2-phénylpropanamide, ou un sel pharmaceutiquement acceptable de celui-ci, ou
le N-t-butyl-2-[[4-(2-méthoxyphényl)pipérazinyl]méthyl]-3-phénylpropanamide, ou un sel pharmaceutiquement acceptable de celui-ci, ou
le 4-(2-méthoxyphényl)-N-(1-méthylcyclohexyl)-1-pipérazinepropanamide, ou un sel pharmaceutiquement acceptable de celui-ci, ou
le 4-(2-pyrimidinyl)-N-(1-méthylcyclohexyl)-1-pipérazinepropanamide, ou un sel pharmaceutiquement acceptable de celui-ci, ou
le (R)-1-méthyl-N-[1-méthyl-2-[4-(2-pyrimidinyl)]-1-pipérazinyl]éthyl]cyclohexanecarboxamide, ou un sel pharmaceutiquement acceptable de celui-ci, ou
le N-(1,1-diméthyléthyl)-3-[4-(2-méthoxyphényl)-1-pipérazinyl]-propanamide, ou un sel pharmaceutiquement acceptable de celui-ci, ou
le N-t-butyl-2-méthoxyphényl-3-(4-(2-méthoxyphénypipérazin-1-yl)propanamide, ou un sel pharmaceutiquement acceptable de celui-ci, ou
le (R*)-N-t-butyl-3-[4-(2-métlioxy)phényl]-pipérazin-1-yl]-2-phénylpropionamide, ou un sel pharmaceutiquement acceptable de celui-ci, ou
le (S∗)-N-t-butyl-3-[4-(2-méthoxy)phényl]pipérazin-1-yl]-2-phénylpropionamide, ou un sel pharmaceutiquement acceptable de celui-ci.

**10.** Procédé de préparation d'un composé selon la revendication 1 qui comprend :

(a) l'acylation d'une amine de formule

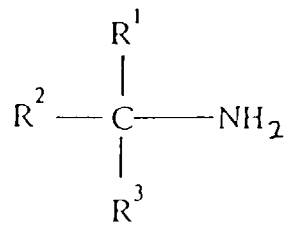

avec un acide de formule

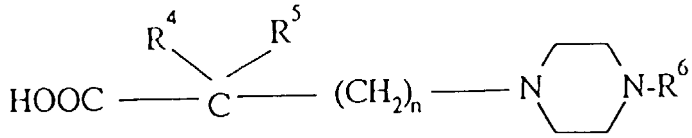

ou avec un dérivé d'acylation de celui-ci

(b) l'alkylation d'une pipérazine de formule

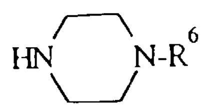

avec un agent d'alkylation fournissant le groupement

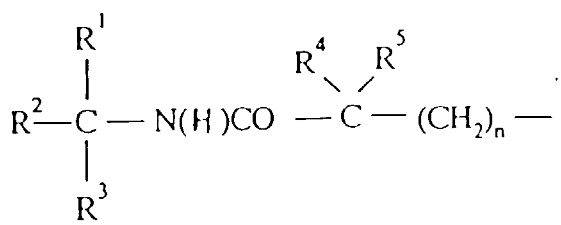

ou

(c) la conversion d'une base libre de formule (I) en un sel pharmaceutiquement acceptable de celle-ci

ou

(d) la résolution d'un composé racémique de formule (I) pour donner un énantiomère.

**11.** Procédé de préparation d'un composé selon la revendication 1 qui comprend la réaction d'un nitrile de formule

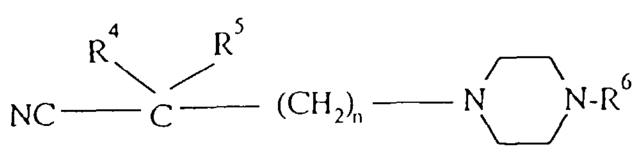

avec un alcool tertiaire de formule

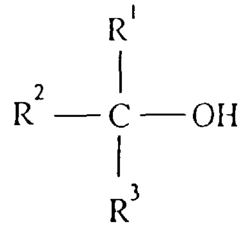

sous des conditions acides.

**12.** Procédé de préparation d'un composé selon la revendication 1 dans lequel $R^4$ est un hydrogène et n est 1 qui comprend la désulfuration d'un composé de formule suivante contenant du soufre

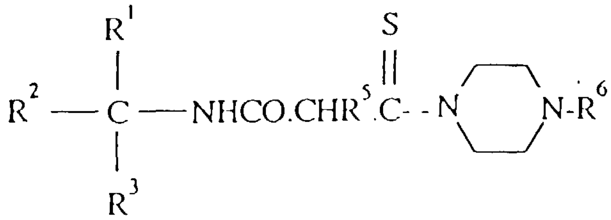

**13.** Composition pharmaceutique comprenant un composé selon l'une quelconque des revendications 1 à 9 en association avec un support pharmaceutiquement acceptable.

**14.** Composé comme décrit dans l'une quelconque des revendications 1 à 9 pour l'utilisation comme produit pharmaceutique.

**Revendications pour les Etats contractants suivants : ES, GR**

**1.** Procédé de préparation d'un composé de formule

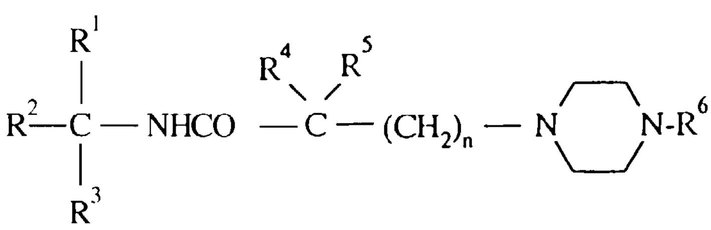

(I)

dans laquelle

$R^1$ est un alkyle de 1 à 6 atomes de carbone ;
$R^2$ et $R^3$ sont des alkyles de 1 à 6 atomes de carbone ou, pris conjointement, forment un polyméthylène de 2 à 12 atomes de carbone ;
$R^4$ est un hydrogène ou un alkyle de 1 à 6 atomes de carbone ;
$R^5$ est un hydrogène, alkyle de 1 à 8 atomes de carbone, hydroxyalkyle de 1 à 3 atomes de carbone, phényle, benzyle, phényle substitué ou benzyle substitué dans lequel les substituants sont hydroxy, halogène, alkyle de 1 à 6 atomes de carbone, alkoxy de 1 à 6 atomes de carbone, trifluorométhyle, nitro, cyano, carbalkoxy

de 2 à 7 atomes de carbone, carboxamido, amino, alkylamino de 1 à 6 atomes de carbone ou dialkylamino de 2 à 12 atomes de carbone ;
$R^6$ est un phényle, benzyle, 2-, 3- ou 4-pyridinyle, 2-pyrimidinyle ou 2-pyrazinyle ; tous pouvant être substitués par un ou plusieurs hydroxy, halogène, alkyle de 1 à 6 atomes de carbone, alkoxy de 1 à 6 atomes de carbone, trifluorométhyle, nitro, cyano, carbalkoxy de 2 à 7 atomes de carbone, carboxamido, amino, alkylamino de 1 à 6 atomes de carbone ou dialkylamino de 2 à 12 atomes de carbone ;
et n est un des entiers 1 ou 2 ; ou un sel pharmaceutiquement acceptable de celui-ci,

qui comprend

(a) l'acylation d'une amine de formule

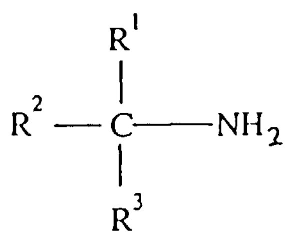

avec un acide de formule

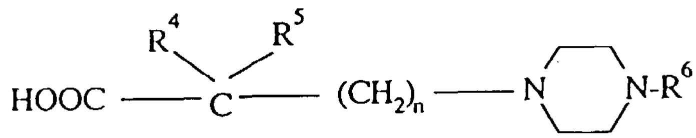

ou avec un dérivé d'acylation de celui-ci
(b) l'alkylation d'une pipérazine de formule

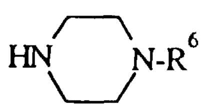

avec un agent d'alkylation fournissant le groupement

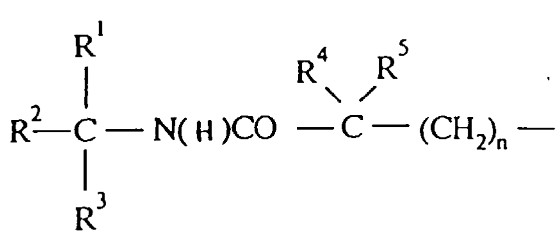

ou
(c) la réaction d'un nitrile de formule

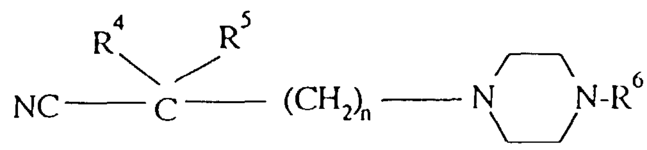

avec un alcool tertiaire de formule

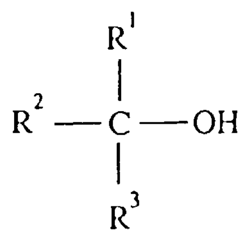

sous des conditions acides

ou

(d) la désulfuration d'un composé de formule suivante contenant du soufre

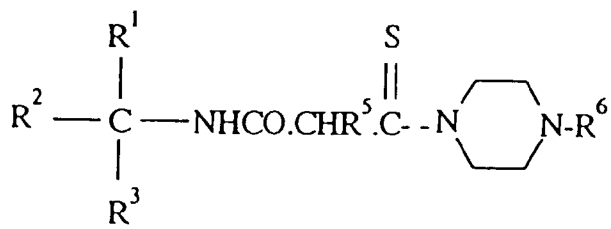

pour donner un composé de formule 1 dans lequel $R^4$ est hydrogène et n est 1 ou
(e) la conversion d'une base libre de formule (I) en un sel pharmaceutiquement acceptable de celle-ci ou
(f) la résolution d'un composé racémique de formule (I) pour donner un énantiomère.

**2.** Procédé selon la revendication 1 dans lequel, dans le produit,

$R^2$ et $R^3$ sont des alkyles de 1 à 6 atomes de carbone ou, pris conjointement, forment un polyméthylène de 2 à 6 atomes de carbone ;
$R^5$ est un hydrogène, alkyle de 1 à 6 atomes de carbone, hydroxyalkyle de 1 à 3 atomes de carbone, phényle, benzyle, p-hydroxyphényle, p-méthoxyphényle, o-méthoxyphényle, p-chlorophényle ou p-fluorophényle ;

et

$R^6$ est un phényle, 2-alkylphényle dans lequel le substituant alkyle a 1 à 6 atomes de carbone, 2-alkoxyphényle dans lequel le substituant alkoxy a 1 à 6 atomes de carbone, 2-halophényle, 2-cyanophényle, 2-nitrophényle, 2-perhalométhylphényle, benzyle, 2-, 3-ou 4-pyridinyle, 2-pyrimidinyle ou 2-pyrazinyle ;

ou un sel pharmaceutiquement acceptable de celui-ci.

**3.** Procédé selon la revendication 1 dans lequel, dans le produit, $R^2$ et $R^3$ sont, indépendamment, des alkyles de 1 à 6 atomes de carbone.

4. Procédé selon la revendication 1 dans lequel, dans le produit, $R^2$ et $R^3$ forment le tétraméthylène, le pentaméthylène ou l'hexaméthylène.

5. Procédé selon l'une quelconque des revendications 1, 3 ou 4 dans lequel, dans le produit, $R^4$ est un hydrogène et $R^5$ est un alkyle de 1 à 6 atomes de carbone, un phényle ou un benzyle.

6. Procédé selon l'une quelconque des revendications 1, 2, 3, 4 ou 5 dans lequel, dans le produit, $R^6$ est le 2-méthoxyphényle, le 2-chlorophényle ou le 2-pyrimidinyle.

7. Procédé selon la revendication 1 dans lequel, dans le produit, $R^5$ est un alkyle de 4 à 8 atomes de carbone, phényle, benzyle, phényle substitué ou benzyle substitué dans lequel les substituants sont halogène, alkyle de 1 à 6 atomes de carbone, alkoxy de 1 à 6 atomes de carbone, trifluorométhyle, nitro, cyano, carbalkoxy de 2 à 7 atomes de carbone, carboxamido, alkylamino de 1 à 6 atomes de carbone ou dialkylamino de 2 à 12 atomes de carbone.

8. Procédé selon la revendication 7 dans lequel, dans le produit, n est 1 et $R^2$ et $R^3$ sont indépendamment, des alkyles de 1 à 6 atomes de carbone.

9. Procédé selon la revendication 1 dans lequel le produit est

   le N-(1,1-diméthyléthyl)-4-(2-pyrimidinyl)-1-pipérazine-propanamide, ou un sel pharmaceutiquement acceptable de celui-ci, ou
   le N-t-butyl-3-[4-(2-méthoxyphényl)pipérazinyl]-2-phénylpropanamide, ou un sel pharmaceutiquement acceptable de celui-ci, ou
   le N-t-butyl-2- [[4-(2-méthoxyphényl)pipérazinyl]méthyl]-3-phénylpropanamide, ou un sel pharmaceutiquement acceptable de celui-ci, ou
   le 4-(2-méthoxyphényl)-N-(1-méthylcyclohexyl)-1-pipérazinepropanamide, ou un sel pharmaceutiquement acceptable de celui-ci, ou
   le 4-(2-pyrimidinyl)-N-(1-méthylcyclohexyl)-1-pipérazinepropanamide, ou un sel pharmaceutiquement acceptable de celui-ci, ou
   le (R)-1-méthyl-N-[1-méthyl-2-[4-(2-pyrimidinyl)]-1-pipérazinyl]éthyl]cyclohexanecarboxamide, ou un sel pharmaceutiquement acceptable de celui-ci, ou
   le N-(1,1-diméthyléthyl)-3-[4-(2-méthoxyphényl)-1-pipérazinyl]-propanamide, ou un sel pharmaceutiquement acceptable de celui-ci, ou
   le N-t-butyl-2-méthoxyphényl-3-(4-(2-méthoxyphénypipérazin-1-yl)propanamide, ou un sel pharmaceutiquement acceptable de celui-ci, ou
   le (R*)-N-t-butyl-3-[4-(2-méthoxy)phényl]-pipérazin-1-yl]-2-phénylpropionamide, ou un sel pharmaceutiquement acceptable de celui-ci, ou
   le (S*)-N-t-butyl-3-[4-(2-méthoxy)phényl]pipérazin-1-yl]-2-phénylpropionamide, ou un sel pharmaceutiquement acceptable de celui-ci.

10. Procédé de préparation d'une composition pharmaceutique comprenant la mise en association d'un composé de formule (I), comme défini dans la revendication 1, ou d'un sel pharmaceutiquement acceptable de celui-ci, avec un support pharmaceutiquement acceptable.

11. Procédé selon la revendication 10 dans lequel l'ingrédient actif est préparé par le procédé revendiqué dans l'une quelconque des revendications 1 à 9.